# EUROPÄISCHE PATENTANMELDUNG

(11) **EP 2 602 020 A2**
(43) Veröffentlichungstag der Anmeldung: **12.06.2013**
(21) Anmeldenummer: 12008183.1
(22) Anmeldetag: 06.12.2012
(51) Int. Cl.: B01F 7/00, A01C 3/02

(54) **Rührvorrichtung für einen Fermenter einer Biogasanlage**

(30) Priorität: 09.12.2011 DE 102011120851
(71) Anmelder: Janovsky, Sebastian, 84544 Aschau am Inn (DE)
(72) Erfinder: Janovsky, Sebastian, 84544 Aschau am Inn (DE)
(74) Vertreter: Liebl, Thomas

(57) **Zusammenfassung**

Die Erfindung betrifft eine Rührvorrichtung für einen Fermenter einer Biogasanlage, mit einem Tauchmotorrührwerk (6) mit einem langgestreckten Rührgerätegehäuse (8) und mit in Längsrichtung herausgeführter Rührerwelle, an der wenigstens zwei Rührflügel (9, 10) angebracht sind. Mit dem Rührgerätgehäuse (8) ist ein senkrecht zur Rührerwelle ausgerichteter Führungsschlitten 12) verbunden, der zu einer Höhenverstellung an einem vertikalen Führungsrohr (7) verschiebbar eingreift. Erfindungsgemäß ist wenigstens ein Führungsschlitten (12, 12') am Rührgerätegehäuse (8) im Längsseitenbereich seitlich angeordnet, so dass die Gesamtanordnung relativ kurz baut.

## Beschreibung

Die Erfindung betrifft eine Rührvorrichtung für einen Fermenter einer Biogasanlage nach dem Oberbegriff des Anspruchs 1.

Eine bekannte, gattungsgemäße Rührvorrichtung eines Fermenters einer Biogasanlage (DE 195 17 901 C1) besteht aus einem Tauchmotorrührwerk mit einem langgestreckten zylindrischen Rührgerätgehäuse mit in Längsrichtung herausgeführter Rührerwelle, an der zwei Rührflügel angebracht sind. Senkrecht zur Rührerwelle ist am Rührgerätgehäuse ein Führungsschlitten angebracht, der zu einer Höhenverstellung des Tauchmotorrührwerks an einem vertikalen Führungsrohr verschiebbar eingreift.

Der Führungsschlitten ist hier an der Rückseite des Rührgerätgehäuses gegenüber liegend zu den Rührflügeln angeordnet, was der Standardanordnung auch bei ähnlichen bekannten Rührvorrichtungen mit Tauchmotorrührwerken entspricht.

Das vertikale Führungsrohr ist um seine Längsachse schwenkbar in einem unteren Schwenklager an der Bodenwand des Fermenters und in einem oberen Schwenklager abgestützt und gehalten. Der obere Bereich des Führungsrohrs ist über eine rechteckige Deckenöffnung des Fermenters nach oben herausgeführt, wobei zur Höhenverstellung des Tauchmotorrührwerks eine handbetriebene Seilwinde am oberen Endbereich des Führungsrohrs angebracht ist, dessen Zugseil nach unten verläuft und mit dem Tauchmotorrührwerk verbunden ist. Damit kann das Tauchmotorrührwerk auch aus der Deckenöffnung heraus in eine Montage- oder Wartungsstellung gezogen werden. In der Betriebsstellung ist die Deckenöffnung mit einer abnehmbaren Abdeckplatte abgedeckt, welche einen Drehteller aufweist, der mit dem Führungsrohr drehfest verbunden ist und der auf einer Ringdichtung gasdicht verdrehbar liegt. Zudem ist durch diesen Drehteller das Zugseil gasdicht mit Stopfbuchsen geführt. Dieser Drehteller beinhaltet auch das obere Schwenklager des Führungsrohrs.

Diese bekannte Rührvorrichtung hat im Wesentlichen folgende Nachteile:
Durch den Führungsschlitten an der Rückseite des Rührgerätgehäuses baut das Tauchrührgerät zusammen mit dem Führungsschlitten relativ lang. Zwischenzeitlich auf dem Markt erhältliche, sehr leistungsstarke und effektiv rührende Tauchmotorrührwerke sind relativ schwer und bereits relativ lang, so dass durch eine rückwärtige Anbringung eines Führungsschlittens der Gesamtaufbau noch länger wird. Um solche Tauchmotorrührwerke in und aus dem Fermenter heben zu können sind entsprechend lange Deckenöffnungen erforderlich, welche hinsichtlich der Statik und des Aufwands ungünstig sind. Insbesondere ist ein Austausch oder eine Nachrüstung bestehender Rührvorrichtungen mit den vorstehenden hoch effizienten Tauchmotorrührwerken ohne eine besonders aufwändige Vergrößerung der Deckenöffnungen nicht möglich.

Ein weiterer Nachteil besteht bei der bekannten Rührvorrichtung darin, dass das obere Stützlager im gasdicht geführten Drehteller angeordnet ist. Die Abstützung der vergleichsweise sehr hohen Schubkräfte und Momente der neuen hoch effizienten Tauchmotorrührwerke in einem Drehteller einer abnehmbaren Abdeckung einer Deckenöffnung ist, wenn überhaupt, allenfalls nur mit einer äußerst aufwändigen Konstruktion möglich.

Aufgabe der Erfindung ist es, eine gattungsgemäße Rührvorrichtung so weiterzubilden, dass die Einbaulänge von Tauchmotorrührwerken, insbesondere von hoch effizienten Tauchmotorrührwerken bei guter Funktion reduziert ist.

Diese Aufgabe wird dadurch gelöst, dass wenigstens ein Führungsschlitten am Rührgerätgehäuse im Längsseitenbereich seitlich angeordnet ist. Die Verbindung des oder der Führungsschlitten mit dem Rührgerätgehäuse kann unmittelbar durch Verschrauben, Schweißen, etc. oder mittelbar über eine gegebenenfalls lösbare Halterung, zum Beispiel eine Klemmhalterung, erfolgen,

Damit ist die Längserstreckung des Tauchmotorrührwerks um die Längsabmessung eines Führungsschlittens reduziert und bei Neuanlagen können Deckenöffnungen mit den bisherigen üblichen Längserstreckungen verwendet werden, was hinsichtlich der Statik und des Herstellungsaufinrands vorteilhaft ist. Ebenso können für Nachrüstungen/Austausch von Tauchmotorrührwerken mit hoch effizienten längeren Tauchmotorrührwerken die bisherigen Deckenöffnungen weiter verwendet werden. Ein weiterer Vorteil dieser Führungsschüttenanordnung besteht darin, dass die vergleichsweise schwereren hoch effizienten Tauchmotorrührwerke in der Nähe ihres Schwerpunkts gut ausbalanciert gehalten sind, was unerwünschten Schwingungen entgegen wirkt. Zudem gleitet durch diese ausbalancierte Anordnung das Tauchmotorrührwerk insgesamt besser und mit weniger Kraftaufwand bei einer Höhenverstellung am Führungsrohr. Dadurch sind die Belastung in den Schwenklagern und insgesamt der Verschleiß reduziert. Grundsätzlich können seitlich am Rührgerätgehäuse mehrere parallel ausgerichtete und jeweils dazugehörigen Führungsrohren zugeordnete Führungsschlitten angeordnet sein. Bevorzugt wird ein einziges Führungsrohr verwendet und ein zugehöriger Führungsschlitten einseitig etwa in der Längsmitte am Rührgerätegehäuse angebracht. Dies ergibt eine kostengünstige Lösung, welche insbesondere auch hinsichtlich gasdichter Durchführungen einer Höhenverstellung und einer Verschwenkbarkeit relativ einfach auszuführen ist.

Bei einer alternativen, je nach den Gegebenheiten gut verwendbaren Ausführungsform sind am Rührgerätgehäuse jeweils gegenüberliegend zwei parallel ausgerichtete Führungsschlitten angebracht, die an einer Doppel-Führungsrohr-Anordnung verschiebbar gehalten sind.

Auch die Rührgerätgehäuse der hoch effizienten Tauchrührgeräte haben regelmäßig im Wesentlichen eine zylindrische langgestreckte Form.

Der Führungsschlitten kann in an sich bekannter Weise einfach als Rohrabschnitt eines Rechteckrohrs oder Quadratrohrs ausgeführt sein, welcher über das Führungsrohr gesteckt wird. Dessen Außendurchmesser entspricht dabei zumindest im Höhenstellbereich abzüglich eines Toleranzfreigangs dem Innendurchmesser des Führungsschlittens. Ein zumindest unrunder Durchmesser des Führungsschlittens und entsprechend des einen Führungsrohrs ist wesentlich, damit das Tauchmotorrührwerk keine unerwünschten Schwenk- und Drehbewegungen um das Führungsrohr ausführt. Bei einer Ausführung mit einem Doppel-Führungsrohr können ersichtlich auch runde Querschnitte verwendet werden.

Eine zweckmäßige Halterung des Rührgerätgehäuses wird damit erreicht, dass an dem oder den Führungsschlitten ein Tragrahmen angebracht ist, welcher zusammen mit dem Führungsschlitten höhenverstellbar ist. In einer Weiterbildung kann bedarfsweise an dem/den Führungsschlitten und/oder am Tragrahmen eines oder mehrere Strömungsleitelemente angebracht sein. Je nach der konkreten Anordnung können solche Strömungsleitelemente im Strömungsbereich hinter dem/den Führungsschlitten oder separat oder zusätzlich im Bereich vor dem/den Führungsschlitten angeordnet sein.

Die seitliche Anordnung des/der Führungsschlitten am Rührgerätgehäuse führt dazu, dass die Länge einer rechteckigen Deckenöffnung nur dem Gesamtlängenmaß des Tauchmotorrührwerks entsprechen muss. Dies bedingt, dass bei einem Führungsrohr dieses entsprechend der Anordnung des einen Führungsschlittens in einem mittleren Längsbereich seitlich in der rechteckigen Deckenöffnung anzuordnen ist. Die Breite der Deckenöffnung ist dabei unproblematisch, da diese durch den erforderlichen Durchtritt der Rührflügel regelmäßig größer als die Breitenerstreckung des Rührgerätgehäuses einschließlich des/der Führungsschlitten ist.

Für eine Verschwenkung des Tauchmotorrührwerks sind bei einem einzigen Führungsrohr jeweils die Rohrenden in einem bodenseitigen Schwenklager und einem oberen Schwenklager abgestützt und gelagert, wobei ein oberer Bereich aus dem Fermenter durch die Deckenöffnung herausragt. Bei der alternativen Ausführung mit einem Doppel-Führungsrohr sind diese beiden Rohre endseitig mit einer Strebe verbunden und mit einem jeweils mittigen unteren und oberen Rohrstutzen verlängert, die entsprechend in einem unteren und oberen Schwenklager gelagert sind.

Eine besonders vorteilhafte Weiterbildung wird darin gesehen, dass bei einer Schwenkmöglichkeit des Tauchrührgeräts ein oberes Schwenklager so weit oberhalb der Deckenöffnung liegt, dass das Tauchmotorrührwerk am Führungsrohr über die Deckenöffnung unter dem Schwenklager herausgehoben werden kann. Dabei ist das Schwenklager über ein Gestell oder ein Gehäuse oder einseitig über einen Galgen am Randbereich der Deckenöffnung stabil abgestützt. Die Stützkräfte am oberen Schwenklager werden somit hier im stabilen Deckenaufbau abgestützt und nicht wie im eingangs angegebenen Stand der Technik in einem Drehteller einer abnehmbaren Öffnungsabdeckung. Eine stabile Abstützung ist gerade für die hohen Schubkräfte und Momente der hoch effizienten Tauchmotorrührwerke wesentlich.

Für die Höhenverstellung kann auch hier in einer an sich bekannten Anordnung eine handbetriebene und/oder motorisch betreibbare Seilwinde am oberen Endbereich des Führungsrohrs oder des Doppel-Führungsrohrs verwendet werden, von der ein Zugseil nach unten zum Tauchmotorrührwerk geführt und dort am Führungsschlitten und/oder am Tragrahmen und/oder am Rührgerätgehäuse befestigt ist. Ebenso ist hier in an sich bekannter Weise die Deckenöffnung betriebsmäßig mit wenigstens einer abnehmbaren Abdeckung, beispielsweise einer Abdeckplatte, gasdicht abzudecken, wobei eine gasdichte Durchführung des Führungsrohrs oder gegebenenfalls eines Rohrstutzens durch die Abdeckung erforderlich ist. Als Dichtmittel für die Durchführung kann vorzugsweise ein gasdicht verdrehbares Drehteil oder gegebenenfalls auch ein verwindbarer Balg verwendet werden. Vorteilhaft ist jedoch hier die Kraftabstützung des oberen Schwenklagers, im Gegensatz zum Stand der Technik, von der gasdichten Durchführung entkoppelt, so dass diese relativ einfach ausgeführt sein kann. Darin kann insbesondere auch die ebenfalls erforderliche gasdichte Durchführung des Zugseils, beispielsweise mit einer Stoffbuchse, integriert sein.

Auch die Schwenkvorrichtung für das um seine Achse schwenkbare Führungsrohr oder um die Rohrstutzen schwenkbare Doppel-Führungsrohr kann im Wesentlichen in der herkömmlichen Art ausgeführt sein, wobei oberhalb der Deckenöffnung ein handbetätigbarer Schwenkhebel oder eine motorisch betätigbare Schwenkeinrichtung mit dem Führungsrohr bzw. Rohrstutzen zusammen wirkt. Zudem ist in an sich bekannter Art eine Arretiereinrichtung für eine eingestellte Schwenkstellung vorzusehen.

Anhand einer Zeichnung wird die Erfindung weiter erläutert.

Es zeigen
- Fig. 1: eine Seitenansicht einer Rührvorrichtung in einem Fermenter einer Biogasanlage,
- Fig. 2a: eine Frontansicht der Rührvorrichtung nach Fig. 1 einer ersten Ausführungsform,
- Fig. 2b: eine entsprechende Frontansicht einer zweiten modifizierten Ausführungsform der Rührvorrichtung,
- Fig. 3: eine Ansicht von oben auf die Rührvorrichtung entsprechend dem Schnitt A-A von Fig. 2a.

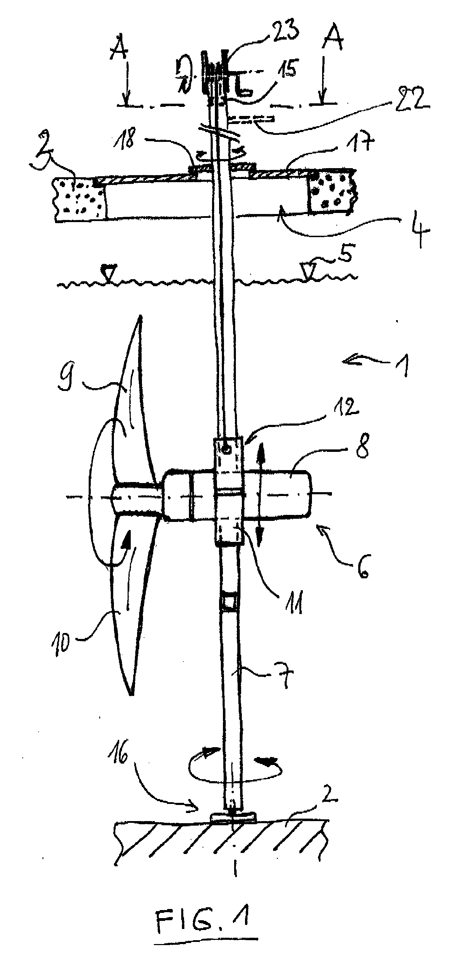

In den Fig. 1 bis 3 sind unterschiedliche Ansichten einer Rührvorrichtung 1 in einem Fermenter einer Biogasanlage dargestellt, von dem ein Teil einer Bodenwand 2 und einer Betondecke 3 mit einer rechteckigen Deckenöffnung 4 sowie das Flüssigkeitsniveau 5 des Substrats dargestellt sind.

Die Rührwerkvorrichtung 1 besteht aus einem Tauchmotorrührwerk 6, welches an einem vertikalen Führungsrohr 7 höhenverstellbar gehalten ist. Das Tauchmotorrührwerk 6 weist ein längliches zylindrisches Rührgerätgehäuse 8 auf mit einer in Längsrichtung herausgeführten Rührerwelle, an der zwei gegenüberstehende Rührflügel 9, 10 befestigt sind. Es handelt sich um ein leistungsstarkes und relativ langes und schweres Tauchmotorrührgerät 6.

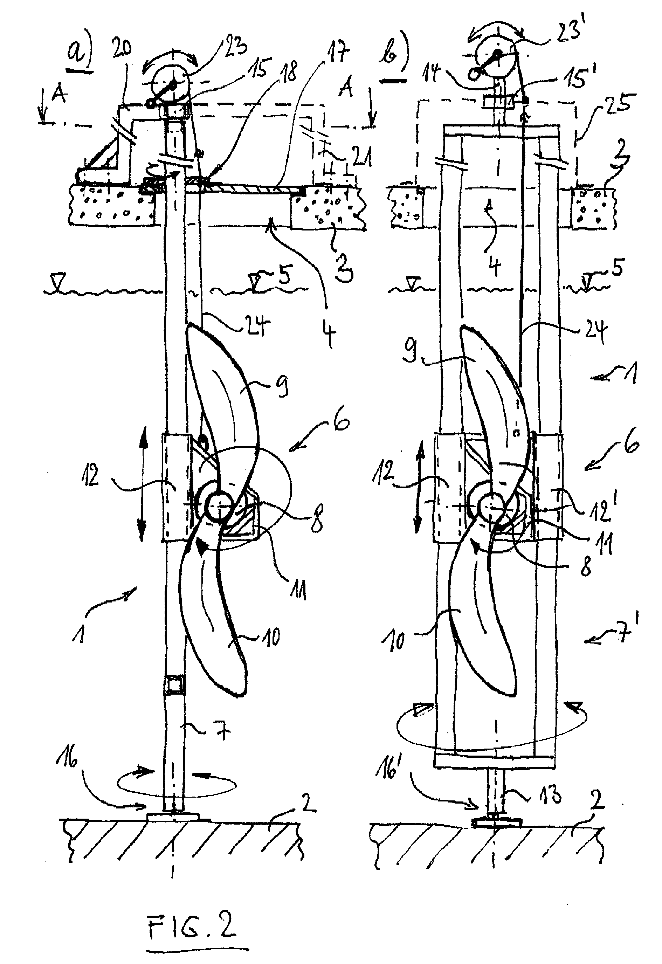

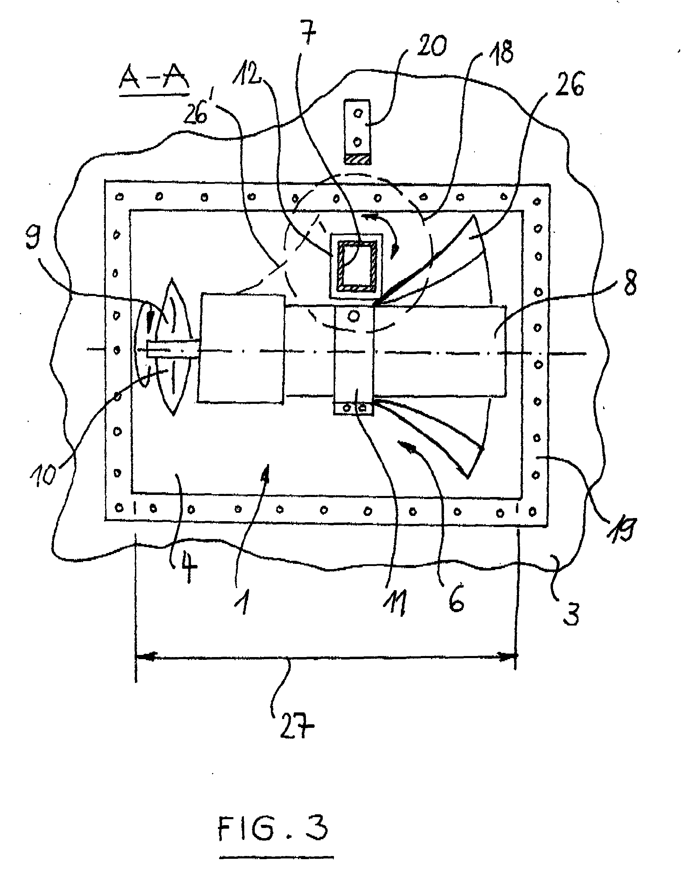

Das Rührgerätgehäuse 8 ist in seinem mittleren Längsbereich über ein Rahmengestell 11 gegebenenfalls mittels einer Klemmeinrichtung mit einem senkrecht zur Rührerwelle, das heißt im eingebauten Zustand vertikal ausgerichteten Führungsschlitten 12 verbunden. Der Führungsschlitten (12) ist ein Rohrabschnitt eines Rechteckrohrs, welcher entsprechend Fig. 2a und Fig. 3 einseitig in der Längsmitte mit dem Rührgerätgehäuse 8 verbunden ist und über ein einziges Führungsrohr 7 für eine verschiebbare Halterung gesteckt ist.

In der modifizierten Ausführungsform nach Fig. 2b sind wiederum im mittleren Längsbereich des Rührgerätgehäuses zwei gegenüber liegende Führungsschlitten 12, 12' angebracht, die entsprechend Fig. 2a aufgebaut sind und die beide über ein Doppel-Führungsrohr 7' gesteckt sind, wobei die beiden Rohre des Doppel-Führungsrohrs parallel verlaufen und in einem oberen und unteren Bereich jeweils mit einer Querstrebe verbunden und in der gemeinsamen Längsachse durch jeweils einen unteren Rohrstutzen 13 und einen oberen Rohrstutzen 14 verlängert sind. Das Führungsrohr 7 sowie das Doppel-Führungsrohr 7' zusammen mit seinem oberen Rohrstutzen 14 sind über die Betondecke 3 hinaus so weit nach oben geführt, dass das Tauchmotorrührwerk 7 an den Führungsschlitten 12, 12' zur Montage oder zu Wartungszwecken aus dem Fermenter herausgezogen werden kann. Entsprechend hoch ist jeweils ein oberes Schwenklager 15 bzw. 15' für eine obere Schwenkabstützung des um seine Längsachse schwenkbaren Führungsrohrs 7 bzw. Doppel-Führungsrohrs 7' angebracht. Entsprechend sind bodenseitig untere Schwenklager 18, 16' angeordnet.

Bei der Ausführungsform mit nur einem Führungsschlitten 12 entsprechend der Fig. 1, 2a und 3 ist der obere Bereich des Führungsrohrs 12 gasdicht aus dem Fermenter herausgeführt, wobei die Deckenöffnung 4 betriebsmäßig durch eine abnehmbare Abdeckplatte 17 gasdicht abgedeckt ist. Die Durchführung des Führungsrohrs 7 durch die Abdeckplatte 17 erfolgt über ein mit dem Führungsrohr 7 verdrehfest verbundenes Drehteil 18, welches gasdicht verdrehbar auf der Abdeckplatte 17 gelagert ist. In Fig. 3 ist die Abdeckplatte 17 abgenommen, so dass nur ein Schachtrahmen 19 zu sehen ist, welcher die rechteckige Abdeckplatte 17 aufnimmt. Zudem ist die Lage des Drehteils 18 strichliert angedeutet.

Das obere Schwenklager 15 ist bei der Ausführung mit nur einem Führungsrohr 7 ersichtlich weit oben an diesem außerhalb des Gasbereichs des Fermenters angeordnet und braucht deshalb nicht gasdicht ausgeführt sein. Aus Fig. 2a ist ersichtlich, dass das Schwenklager 15 mit einer stabilen Galgenanordnung 20 abgestützt ist, welche im Bedarfsfall zur Erhöhung der Stabilität zu einem (strichliert) angedeuteten Gestell 21 ergänzt werden kann. Für die horizontale Verschwenkung des Tauchmotorrührwerks 16 durch eine Verdrehung des Führungsrohrs 7 ist lediglich strichliert ein Handhebel 22 angedeutet, welcher mit einer (nicht dargestellten) Arretiervorrichtung zusammenwirkt.

Oberhalb des Schwenklagers 15 ist am Führungsrohr 7 zudem eine Seilrolle 23 angeordnet, von der ein Zugseil 24 nach unten durch eine gasdichte Durchführung im Drehteil 18 zum Rahmengestell 11 führt. Durch Betätigung der Seilroile 23 kann somit das Tauchrührgerät 6 unterhalb des Flüssigkeitsniveaus 5 betriebsmäßig höhenverstellt werden und bei Bedarf, insbesondere zu Wartungszwecken, aus der rechteckigen Deckenöffnung 4 herausgehoben werden.

Bei der modifizierten Ausführungsform nach 2b ist das Schwenklager 15' in einen mittleren Bereich eines Gehäuses 25 (strichliert angedeutet) angeordnet, welches die Deckenöffnung 4 gasdicht abdeckt. Auch hier ist über dem Gehäuse 25 am oberen Rohrstutzen 14 eine Seilrolle 23' angebracht, dessen Zugseil 24 gasdicht durch das Gehäuse 25 geführt ist und nach unten zu einer Befestigung am Rahmengestell 11 führt.

In Fig. 3 sind zudem am Rahmengestell 11 sich nach hinten erweiternde Strömungsleitelemente 26 angebracht, deren Form und Größe auf die jeweils vorliegenden Gegebenheiten abzustimmen ist. Gegebenenfalls kann es auch zweckmäßig sein am Rührgerätgehäuse ein (strichliert eingezeichnetes) Strömungsleitelement 26' dergestalt anzubringen, dass die Strömung am seitlich liegenden Führungsschlitten 12 bzw. am Führungsrohr 7 vorbeigeführt wird.

Insbesondere aus Fig. 3 ist ersichtlich, dass durch die seitliche Anbringung des Führungsschlittens 12 oder der beiden Führungsschlitten 12, 12' am Rührgerätgehäuse 8 die Gesamtlänge des Tauchmotorrührwerks 6 wesentlich kürzer ist im Vergleich zur bisher üblichen Anbringung eines Führungsschlittens hinten am Rührgerätgehäuse 8. Entsprechend kann damit die Länge 27 der rechteckigen Deckenöffnung 4 vorteilhaft relativ kurz gehalten werden bzw. können vorhandene relativ kurze Deckenöffnungen 4 weiter verwendet werden, wenn vorhandene Tauchmotorrührwerke gegen längere, hoch effiziente Tauchmotorrührwerke ausgetauscht werden, wobei dann lediglich die Lage des Führungsrohrs 7 anzupassen ist.

## Patentansprüche

1. Rührvorrichtung für einen Fermenter einer Biogasanlage,
mit einem Tauchmotorrührwerk (6) mit einem langgestreckten Rührgerätgehäuse (8) mit in Längsrichtung herausgeführter Rührerwelle, an der wenigstens zwei Rührflügel (9, 10) angebracht sind, und
mit einem senkrecht zur Rührerwelle ausgerichteten Führungsschlitten (12), der mit dem Rührgerätgehäuse (8) verbunden ist und der zu einer Höhenverstellung an einem vertikalen Führungsrohr (7) verschiebbar eingreift,
**dadurch gekennzeichnet,**
**dass** wenigstens ein Führungsschlitten (12, 12') am Rührgerätgehäuse (8) im Längsseitenbereich seitlich angeordnet ist.

2. Rührvorrichtung nach Anspruch 1, **dadurch gekennzeichnet, dass** ein einem Führungsrohr (7) zugeordneter Führungsschlitten (12) einseitig oder zwei parallel ausgerichtete, einem Doppel-Führungsrohr (7') zugeordnete Führungsschlitten (12, 12') beidseitig am Rührgerätgehäuse (8) angeordnet sind.

3. Rührvorrichtung nach Anspruch 1 oder 2, **dadurch gekennzeichnet, dass** das Rührgerätgehäuse (8) eine im Wesentlichen zylindrische Form aufweist.

4. Rührvorrichtung nach einem der Ansprüche 1 bis 3, **dadurch gekennzeichnet, dass** der Führungsschlitten (12, 12') ein Rohrabschnitt, insbesondere eines Rechteckrohrs oder Quadratrohrs ist, welcher über das zugeordnete Führungsrohr (7, 7') gesteckt ist, welches zumindest im Höhenstellbereich mit seinem Außendurchmesser abzüglich eines Toleranzfreigangs dem Innendurchmesser des Führungsschlittens (12, 12') entspricht.

5. Rührvorrichtung nach Anspruch 4, **dadurch gekennzeichnet, dass** am Führungsschlitteri (12, 12') ein Tragrahmen als Rahmengestell (11) angebracht ist, in dem das Rührgerätgehäuse (8) gehalten ist, und
dass bedarfsweise am Führungschlitten (12, 12') und/oder am Tragrahmen (11) und/oder am Rührgerätgehäuse (8) Strömungsleitelemente (26, 26') angebracht sind.

6. Rührvorrichtung nach einem der Ansprüche 1 bis 5 eines Fermenters mit einer Bodenwand (2) und einer Decke, insbesondere als Betondecke (3) oder als Foliendach mit einer rechteckigen Deckenöffnung (4) als Durchführungsöffnung für das Tauchmotorrührwerk (6), wobei jeweils die Rohrenden bei einem Führungsrohr (7) oder jeweils ein Rohrstutzen (13, 14) an einer oberen und unteren Verbindung eines Doppel-Führungsrohrs (7') in einem bodenseitigen unteren Schwenklager (16, 16') und in einem oberen Schwenklager (15, 15') abstützt und gelagert ist, wobei ein oberer Bereich aus dem Fermenter durch die Deckenöffnung (4) herausragt, **dadurch gekennzeichnet, dass** bei einem Führungsrohr (7) dieses in einem mittleren Längsbereich seitlich in der rechteckigen Deckenöffnung (4) und bei einem Doppel-Führungsrohr (7) dessen oberer Rohrstutzen (14) in der Mitte der Deckenöffnung (4) angeordnet ist.

7. Rührvorrichtung nach Anspruch 6, **dadurch gekennzeichnet, dass** die Deckenöffnung (4) betriebsmäßig mit wenigstens einer Abdeckung, insbesondere als Abdeckplatte (17) gasdicht abdeckbar ist, und
dass für eine gasdichte Durchführung des Führungsrohrs (7) durch die Abdeckung (17) dort ein Dichtmittel als gasdichtes Drehteil (18) oder ein verwindbarer Balg angeordnet ist.

8. Rührvorrichtung nach Anspruch 6 oder 7, **dadurch gekennzeichnet, dass** das obere Schwenklager (14) oberhalb der Deckenöffnung (4) so hoch angeordnet ist, dass das Tauchmotorrührwerk (6) am Führungsrohr (7, 7') über die Deckenöffnung (4) unter dem oberen Schwenklager (15, 15') heraushebbar ist, und
dass das obere Schwenklager (15, 15') über ein Gestell oder einseitig über einen Galgen (20) oder ein Gehäuse (26) am Randbereich der Deckenöffnung (4) abgestützt ist.

9. Rührvorrichtung nach einem der Ansprüche 6 bis 8, **dadurch gekennzeichnet, dass** zur Höhenverstellung eine handbetriebene und/oder motorisch betreibbare Seilwinde (23, 23') am oberen Endbereich des Führungsrohrs (7) oder des Doppel-Führungsrohrs (7') angebracht ist, von der ein Zugseil (24) nach unten zum Tauchmotorrührwerk (6) geführt ist und dort am Führungsschlitten (12, 12') und/oder am Tragrahmen (11) und/oder am Rührgerätgehäuse (8) befestigt ist, und dass das Zugseil (24) gasdicht durch die Abdeckung (17) der Deckenöffnung (4) geführt ist.

10. Rührvorrichtung nach Anspruch 9, **dadurch gekennzeichnet, dass** die gasdichte Durchführung des Zugseils (24) durch das gasdichte Drehteil (18), vorzugsweise über eine Stopfbuchsenanordnung erfolgt.

11. Rührvorrichtung nach einem der Ansprüche 6 bis 10, **dadurch gekennzeichnet, dass** als Schwenkvorrichtung für das um seine Achse schwenkbare Führungsrohr (7) oder Doppel-Führungsrohr (7') oberhalb der Deckenöffnung (4) ein handbetätigbarer Schwenkhebel (22) und/oder eine motorisch betätigbare Schwenkeinrichtung in Verbindung mit einer Arretiereinrichtung für eine eingestellte Schwenkstellung vorgesehen ist.
